Europäisches Patentamt

European Patent Office

Office européen des brevets

(19)

(11) Veröffentlichungsnummer: **0 158 196**

**A2**

(12) ## EUROPÄISCHE PATENTANMELDUNG

(21) Anmeldenummer: 85103427.2

(22) Anmeldetag: 22.03.85

(51) Int. Cl.⁴: **C 07 C 45/50**
**C 07 C 47/02**

(30) Priorität: 03.04.84 DE 3412336

(43) Veröffentlichungstag der Anmeldung:
16.10.85 Patentblatt 85/42

(84) Benannte Vertragsstaaten:
DE FR GB IT NL SE

(71) Anmelder: Ruhrchemie Aktiengesellschaft
Bruchstrasse 219
D-4200 Oberhausen 11(DE)

(72) Erfinder: Lieder, Bernhard
Siegfriedstrasse 61
D-4250 Bottrop(DE)

(72) Erfinder: Schmidt, Volkmar, Dipl.-Ing.
Lützowstrasse 52
D-4200 Oberhausen 11(DE)

(72) Erfinder: Sedelies, Sylvia, Dipl.-Ing.
Deutsch-Luxemburgerstrasse 151
D-4600 Dortmund 50(DE)

(72) Erfinder: Kalbfell, Heinz, Dipl.-Ing.
Eichenstrasse 20
D-4235 Schermbeck(DE)

(74) Vertreter: Reichelt, Karl-Heinz, Dr.
m. Br. Ruhrchemie Aktiengesellschaft Abt. PLD Postfach
13 01 60
D-4200 Oberhausen 13(DE)

(54) Verfahren zur Hydroformylierung von Propylen.

(57) Aus dem bei der Hydroformylierung von Propylen anfallenden Abgas wird nicht umgesetztes Propylen durch partielle Kondensation abgetrennt. Das Kondensat wird rektifiziert, die Propylenfraktion in die Reaktionsstufe zurückgeführt.

EP 0 158 196 A2

Ruhrchemie Aktiengesellschaft, Oberhausen 11

## Verfahren zur Hydroformylierung von Propylen

Die Erfindung betrifft ein Verfahren zur Hydroformylierung von Propylen unter niederem Druck. Hierbei wird das Abgas der Reaktion, das noch erhebliche Anteile nicht umgesetztes Olefin enthält, in mehreren Stufen partiell kondensiert und das erhaltene Kondensat zur Rückgewinnung des Propylens rektifiziert.

Die Hydroformylierung von Olefinen im großtechnischen Maßstab erfolgt im wesentlichen nach drei Prozessen. Beim Hochdruck-Verfahren (HD-Verfahren) werden die Einsatzolefine je nach Zahl der Kohlenstoffatome des Olefins und je nach gewünschtem Reaktionsprodukt, d.h. Aldehyde oder Alkohole, bei Drücken von 100 bis 350 bar ($10 \cdot 10^3$ bis $35 \cdot 10^3$ kPa) und Temperaturen von 120 bis 180°C mit Synthesegas umgesetzt. Aktiver Katalysator der Reaktion ist Hydridokobalttetracarbonyl, das als solches eingesetzt oder während der Reaktion aus geeigneten Kobaltverbindungen gebildet werden kann. Diese HD-Verfahren sind unter entsprechenden Bedingungen im allgemeinen durch hohe Olefinumsätze gekennzeichnet. Das Abgas der HD-Reaktion enthält neben wenig Einsatzolefin vor allem das entsprechende Paraffin und Synthesegas. Auf Maßnahmen zur Olefinrückgewinnung und -rückführung kann daher verzichtet werden.

Beim Mitteldruck-Verfahren (MD-Verfahren) werden Kobalt-
Phosphin-Komplexverbindungen als Katalysatoren verwendet.
Der niedrige Reaktionsdruck von 50 bis 100 bar (5 · $10^3$
bis 10 · $10^3$ kPa) hat u.a. eine Senkung des Olefinumsatzes
zur Folge. Die durch Phosphine modifizierten Kobaltkatalysatoren wirken unter den angewendeten Arbeitsbedingungen
stark hydrierend; daher ist das nicht umgesetzte Olefin
aus der Reaktion im allgemeinen erheblich mit Paraffin
der gleichen Kohlenstoffatomzahl verunreinigt. Weil der
Wiedereinsatz des Olefins eine kostspielige Olefin/
Paraffintrennung erfordert, wird auf eine Olefingewinnung
und -rückführung und damit eine wirtschaftlich optimale
Verfahrensführung meistens verzichtet.

Im Niederdruck-Verfahren (ND-Verfahren) schließlich werden
durch Phosphin modifizierte Rhodiumkomplexverbindungen als
Katalysatoren eingesetzt. Die Hydroformylierung erfolgt bei
Temperaturen von 60 bis 150°C und Drücken von 10 bis 80 bar
( 1 · $10^3$ bis 8 · $10^3$ kPa). Der Vorteil des ND-Verfahrens
besteht vor allem in der hohen Selektivität der Hydroformylierungsreaktion.

Der Nachteil aller ND-Verfahren ist, daß die tiefen Reaktionstemperaturen bei Einsatz von Propylen technischer Qualität, das bis zu 6 Gew.% Propan enthält, nur einen begrenzten Olefinumsatz erlauben, so daß aus wirtschaftlichen Gründen das Einsatzolefin wiedergewonnen und zurückgeführt werden
muß. Dem steht der Vorteil gegenüber, daß die durch Liganden
modifizierten Rhodiumkatalysatoren wesentlich geringer
hydrierend wirken als die Kobalt-Phosphin-Katalysatoren. Daher ist die Bildung von Paraffin aus dem Einsatzolefin geringer als bei der MD Verfahren. Aber auch bei der ND-Oxo-

- 3 -

EP0158196

Synthese mit kontinuierlicher Wiedergewinnung und kontinuierlichem Wiedereinsatz des je Reaktordurchgang nicht umgesetzten Einsatzolefins muß eine Olefin-Verwertung vorgesehen werden. Bei Verzicht auf eine derartige Verwertung gehen mit dem für die Oxostufe wertlosen Paraffin unverhältnismäßig hohe Olefinanteile verloren.

Die aufgezeigten Schwierigkeiten können dadurch behoben werden, daß das technische Propylen durch Refraktionierung gereinigt und auf einen Gehalt von mindestens 99 Gew.% Propylen angereichert wird.

Eine andere Möglichkeit, das Propylen unter weitgehender Vermeidung von Verlusten umzusetzen besteht in der absorptiven Abtrennung des Olefins aus dem Abgas und seiner Rückführung in die Reaktionsstufe nach Desorption. Schließlich kann das restliche Propylen auch in ND-Anlagen, die dem Hauptreaktor als Kaskade nachgeschaltet sind, vollends in Butyraldehyd überführt werden.

Die vorstehend geschilderten Schwierigkeiten treten nicht nur bei Einsatz von Propylen technischer Qualität mit hohem Propangehalt auf, sondern auch bei der Verwendung von Synthesegas mit entsprechend hohem Inertenanteil.

Alle vorstehend beschriebenen Varianten zur Minderung der Propylenverluste erweisen sich für technische Anlagen als unwirtschaftlich.

Es bestand daher die Aufgabe, eine Arbeitsweise zu entwickeln, die es gestattet, im Abgas von Niederdruck-Hydroformylierungs-Anlagen zur Herstellung von Butyraldehyd enthaltenes nicht umgesetztes Propylen abzutrennen.

Die vorstehend geschilderte Aufgabe wird gelöst durch ein Verfahren zur Hydroformylierung von Propylen bei 20 bis 150°C und 1 bis 200 bar (100 bis 20 · $10^3$ kPa) in Gegenwart von Rhodiumkatalysatoren. Hierbei wird das nicht umgesetztes Propylen enthaltende Abgas der Reaktionsstufe partiell kondensiert, das Kondensat rektifiziert und die Propylenfraktion in die Reaktionsstufe zurückgeführt.

Überraschenderweise gelingt es nach dem erfindungsgemäßen Verfahren, auch Propylen lediglich technischer Reinheit annähernd verlustfrei in Butyraldehyd zu überführen.

Ein typisches Abgas, das bei der Hydroformylierung von Propylen anfällt, enthält 25 bis 40 Vol.% Propylen, 6 bis 20 Vol.% Propan, 20 bis 40 Vol.% Wasserstoff und 10 bis 40 Vol.% Kohlenmonoxid. Es verläßt den Reaktor mit einer Temperatur von 60 bis 150°C und einem Druck von 10 bis 80 bar (1 · $10^3$ bis 8 · $10^3$ kPa).

Entsprechend dem neuen Prozeß wird das Abgas ohne weitere Vorbehandlung soweit abgekühlt, daß sich unter den gegebenen Druckbedingungen das Propylen weitgehend kondensiert. Die Abkühlung erfolgt in gängigen Kondensatoren. Als Kühlmittel finden Einsatzolefin, Kühlwasser und übliche Kältemittel, wie Sole, Anwendung. Die Temperatur und die Menge des Kühlmittels richten sich nach der Menge und Temperatur und Druck des Abgases.

Nach einer bevorzugten Ausführungsform des erfindungsgemäßen Verfahrens wird die Kondensation nicht ein-, sondern mehrstufig durchgeführt. Zweckmäßig ist es hierbei in der ersten Stufe, etwa 10 Vol.% des Propylens und den Rest in weiteren Stufen zu kondensieren. Die Zahl der Stufen richtet sich nach Propylen-, Propan- und Aldehydpartialdruck.

Bei hohem Propylenpartialdruck sind weniger Kondensationsstufen erforderlich, als bei niedrigem Partialdruck des Olefins. Bewährt hat es sich, mit 2 bis 5 Kondensationsstufen zu arbeiten. Das Kühlmittel kann in den einzelnen Stufen gleiche Temperatur aufweisen, zweckmäßig arbeitet man aber mit Kühlmittel abnehmender Temperatur in den aufeinanderfolgenden Stufen. Selbstverständlich ist es auch möglich, verschiedene Kühlmittel in den einzelnen Stufen einzusetzen.

Das nach Abtrennung des Propylens verbleibende Restgas besteht im wesentlichen aus Propan, Wasserstoff und Kohlenmonoxid. Es wird im allgemeinen verbrannt.

Zur Gewinnung reinen Propylens wird das Kondensat rektifiziert und die dabei mit einer Reinheit entsprechend dem Einsatzolefin anfallende Propylenfraktion in die Reaktionsstufe zurückgeführt.

Die Rektifikation erfolgt zweckmäßig in einer Kolonne mit 60 bis 80 Böden. Druck und Temperatur können unter Beachtung der kritischen Werte des Propylens variiert werden. Besonders bewährt hat es sich, bei Kopftemperaturen von 40 bis 60°C und bei Drücken von 15 bis 25 bar ($1,5 \cdot 10^3$ bis $2,5 \cdot 10^3$ kPa) zu arbeiten. Dabei ist darauf zu achten, daß die Temperatur im Sumpf ausreichend unterhalb der Zersetzungstemperatur des Butyraldehyds liegt.

Bei mehrstufiger Kondensation wird mit Erfolg in der Weise gearbeitet, daß man aus dem Abgas eine propylenarme (entsprechend aldehydreiche) und eine propylenreiche (entsprechend aldehydarme) Fraktion abtrennt oder mehrere abgetrennte Fraktionen zu einer propylenarmen und einer propylenreichen Teilfraktion vereinigt und diese Teilfraktionen getrennt in zwei Zuläufen in den unteren bzw. oberen Teil der Kolonne aufgibt. Am Kopf der Kolonne erhält man Propylen einer Reinheit entsprechend dem Einsatzolefin oder höher.

In einer, der ersten Kolonne nachgeschalteten zweiten Kolonne werden aus dem Destillationsrückstand der ersten Kolonne noch Propan und Butyraldehyd gewonnen.

Zur Beheizung der Kolonnen verwendet man mit Erfolg den unter Nutzung der Reaktionswärme erzeugten Dampf.

Das erfindungsgemäße Verfahren eignet sich insbesondere zur Aufarbeitung von Abgasen, wie sie bei der Hydroformylierung von Propylen mit wasserlöslichen Katalysatoren in einem aus wässriger und organischer Phase bestehenden Zweiphasensystems erhalten werden. Derartige Katalysatoren sind bekannt. Es handelt sich hierbei um Rhodiumkomplexverbindungen, deren Wasserlöslichkeit durch die Anwesenheit sulfonierter oder carboxylierter Arylphosphine als Komplexbestandteil erreicht wird. Die Abtrennung des Katalysators vom Reaktionsprodukt nach Beendigung der Hydroformylierungsreaktion erfolgt bei diesem Verfahren einfach durch Trennung von wässriger und organischer Phase. Zur Gewinnung der Wertprodukte, n-Butyraldehyd neben wenig i-Butyraldehyd, wird das Reaktionsgemisch destilliert. Das in der Reaktionsstufe anfallende Abgas wird nach dem erfindungsgemäßen Verfahren aufgearbeitet. Durch Anwendung des beanspruchten Prozesses läßt sich der Propylenumsatz von 85 bis 90 % auf über 97 % steigern.

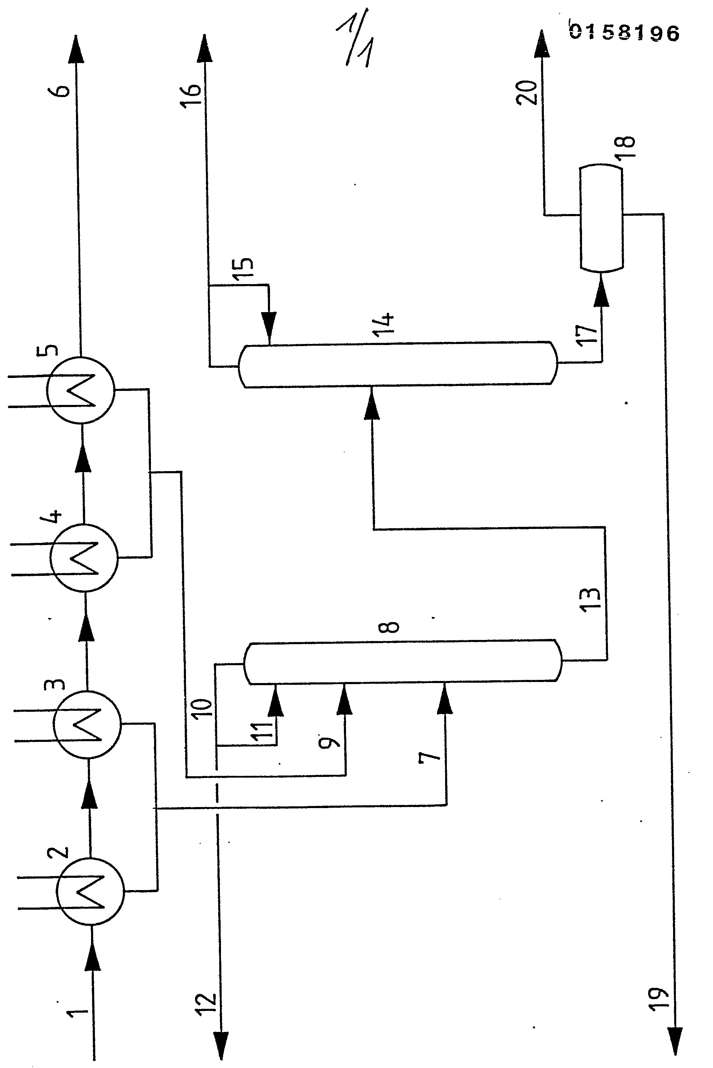

In der beigefügten Zeichnung wird das Verfahren näher erläutert.

Abgas aus der Reaktionsstufe gelangt über eine Leitung 1 in Wärmeaustauscher 2, 3, 4 und 5 und wird hier partiell kondensiert. Das Restgas wird über eine Leitung 6 der Verbrennung zugeführt.

Die in den Wärmeaustauschern 2 und 3 anfallenden Kondensatströme werden vereinigt über eine Leitung 7 dem unteren und die in den Wärmeaustauschern 4 und 5 anfallenden Kondensatströme über eine Leitung 9 dem oberen Teil einer Rektifikationskolonne 8 zugeführt.

Von dem über eine Leitung 10 abgeführten Destillat wird mittels einer Leitung 11 Kolonnenrücklauf abgezweigt. Propylen als Kopfprodukt gelangt über eine Leitung 12 zurück zur Reaktionsstufe. Das Sumpfprodukt wird über eine Leitung 13 einer Rektifikationskolonne 14 zugeführt und dort getrennt.

Das Kopfprodukt der Rektifikationskolonne 14, Propan, wird nach Abtrennung des Rücklaufs über eine Leitung 15 durch eine Leitung 16 der weiteren Verwendung zugeleitet. Das Sumpfprodukt wird aus der Rektifikationskolonne über eine Leitung 17 abgezogen. In einem Dekantiergefäß 18 trennt es sich in eine wässrige und eine organische Phase auf.

Die Wasserphase wird über eine Leitung 19 in die Synthesestufe zurückgefahren, die den Rohaldehyd enthaltende organische Phase wird über eine Leitung 20 der weiteren Aufarbeitung zugeführt.

Oberhausen 11, 29.03.1984  0158196
PLD rcht-sei    R 1966

Ruhrchemie Aktiengesellschaft, Oberhausen 11

Patentansprüche

1.) Verfahren zur Hydroformylierung von Propylen bei
20 bis 150°C und 1 bis 200 bar (100 bis 20 · 10³ kPa) in
Gegenwart von Rhodiumkatalysatoren, dadurch gekennzeichnet,
daß das nicht umgesetzte Propylen enthaltende Abgas der
Reaktionsstufe partiell kondensiert, das Kondensat rektifiziert und die Propylenfraktion in die Reaktionsstufe
zurückgeführt wird.

2.) Verfahren nach Anspruch 1, dadurch gekennzeichnet,
daß die Kondensation mehrstufig durchgeführt wird.

3.) Verfahren nach Anspruch 1 und 2, dadurch gekennzeichnet,
daß man aus dem Abgas eine propylenarme und eine propylenreiche Fraktion abtrennt und die propylenarme in den unteren
und die propylenreiche in den oberen Teil der Kolonne
aufgibt.

1/1

0158196